# EUROPEAN PATENT APPLICATION

(11) **EP 1 489 420 A1**
(43) Date of publication of application: **22.12.2004**
(21) Application number: 03743624.3
(22) Date of filing: 06.03.2003
(51) Int. Cl.: G01N 33/543, G01N 33/553, G01N 33/53

(54) **INSTRUMENTS FOR DETECTING LOW-MOLECULAR WEIGHT SUBSTANCE**

(30) Priority: 07.03.2002 JP 2002062050; 18.10.2002 JP 2002304709
(71) Applicant: Enbiotec Laboratories Co., Ltd., Koto-ku, Tokyo 135-8073 (JP)
(72) Inventor: MIZUKAMI, Haruki, ENBIOTEC LABORATORIES CO., LTD., Koto-ku, Tokyo 135-8073 (JP); NISHI, Kazuto, ENBIOTEC LABORATORIES CO., LTD., Koto-ku, Tokyo 135-8073 (JP)
(74) Representative: Bohmann, Armin K., Dr.
(86) International application number: PCT/JP2003/002657
(87) International publication number: WO 2003/075011

(57) **Abstract**

To provide the following instruments 1 and 2 as a low-molecular-weight substance detection instrument employing immunochromatography capable of detecting conveniently and sensitively detecting a low-molecular weight substance such as an environmental pollutant (e.g., a dioxin), as a target substance contained in a test sample:
1. an instrument, which comprises 1) a test sample application section with which a test sample is brought into contact; 2) a label product reaction section containing a label product containing, as a portion thereof, an antibody capable of binding to a target substance contained in the test sample, the label product being not bound to the reaction section; 3) an unbound label product capturing section containing an element capable of capturing the label product which is not bound to the target substance, the element being bound to the capturing section; and 4) a detection section containing a detection element which, when coming into contact with the target substance bound to the label product, causes a visually observable change, and
2. an instrument wherein a test sample is reacted with a labeled antibody containing, as a portion thereof, an antibody capable of binding to a target substance contained in the test sample, and the resultant reaction product is employed for detecting the target substance contained in the test sample.

## Description

### Technical Field

The present invention relates to an instrument for detecting a specific substance.

### Background Art

In recent years, environmental pollutants such as dioxins and PCBs have invited serious problems in terms of environmental pollution, raising concerns about their adverse effects on living organisms, including humans. Conventionally, environmental pollutants such as dioxins have generally been detected by means of gas chromatography-mass spectrometry (GC-MS). GC-MS, which is employed as an official analytical method, provides excellent sensitivity and accuracy. However, GC-MS requires a special apparatus, and therefore involves problems; for example, high analysis cost and requirement for an intricate analysis procedure.

In an attempt to solve the aforementioned problems associated with GC-MS, generally, environmental pollutants such as dioxins have been detected by means of ELISA employing antibodies to these chemical substances, which is a more convenient method than GC-MS.

Although ELISA provides excellent quantification and exhibits high sensitivity, it involves, for example, the following problems: a special apparatus is generally required for detection of a sample, and, when such an apparatus is not employed, sample detection requires a long period of time (i.e., one hour or more). In view of the foregoing, immunochromatography has become of interest, by virtue of its simple operation, high sensitivity, and short detection time.

Among a variety of modes of ELISA, which has been most widely employed in immunochromatography, the sandwich method is frequently performed. The sandwich method employs two different antibodies capable of binding to different sites of a high-molecular-weight analyte substance (substance which *per se* has antigenicity) (see, for example, Japanese Patent Application Laid-Open (*kokai*) Nos. 10-62420 and 2001-124771). In a detection instrument employing the sandwich method, development color occurs to a degree in proportion to the concentration of the high-molecular-weight substance (target substance), whereby the target substance is readily detected. Therefore, such a detection instrument is widely employed in, for example, clinical laboratory tests performed in hospitals, and pregnancy tests performed at home.

When the target substance is a low-molecular-weight compound such as a dioxin or PCB, the sandwich method is difficult to apply to the target substance, since the low-molecular-weight compound and the antibodies differ greatly in molecular weight. Therefore, when such a low-molecular-weight compound is detected by means of immunochromatography, conceivably, it is desirable to employ the competitive method, in which the low-molecular-weight compound (target substance), and a low-molecular-weight compound or a compound similar to the target low-molecular-weight compound are competitively bound to an antibody, followed by detection of the target low-molecular-weight compound.

However, when the competitive method is employed, since the degree of coloring that is inversely proportional to the concentration of the target low-molecular-weight compound is necessarily used as an index, detection of the target substance becomes burdensome, as compared with the case of the sandwich method, in which the degree of coloring that is proportional to the concentration of the target substance is used as an index. Therefore, immunochromatography employing the competitive method has not yet prevailed, and involves difficulty in detecting low-molecular-weight environmental pollutants such as dioxins and PCBs.

### Disclosure of the Invention

The present inventors have found that the aforementioned problems can be solved by providing an instrument for detecting a low-molecular-weight substance (hereinafter may be referred to as a "low-molecular-weight substance detection instrument"), the instrument employing immunochromatography, which instrument comprises a test sample application section with which a test sample is brought into contact, wherein a target substance contained in the test sample brought into contact with the test sample application section is detected by use, as an index, of a labeling substance which forms a label product containing, as a portion thereof, an antibody capable of binding to the target substance contained in the test sample (hereinafter the instrument may be referred to as "the present detection instrument").

The target substance contained in the test sample, which is to be detected by the present detection instrument, is an artificial low-molecular-weight chemical substance which is present in a trace amount in the living environment (hereinafter such a substance may be collectively called an "environmental pollutant"). Particularly appropriate examples of the environmental pollutant include substances suspected of having endocrine disrupting effect, which are so-called "endocrine-disrupting chemicals." Specific examples include dioxins, polychlorinated biphenyls (PCBs), polybrominated biphenyls (PBBs), hexachlorobenzene (HCB), pentachlorophenol (PCP), 2,4,5-trichlorophenoxyacetic acid, 2,4-dichlorophenoxyacetic acid, amitrole, atrazine, alachlor, CAT (simazine), hexachlorocyclohexane, ethyl parathion, NAC (carbaryl), chlordane, oxychlordane, trans-nonachlor, 1,2-dibromo-3-chloropropane, DDT (dichlorodiphenyltrichloroethane), DDE (dichlorodiphenyltrichloroethylene), DDD (dichlorodiphenyldichloroethane), kelthane, aldrin, endrin, dieldrin, endosulfan (benzoepin), heptachlor, heptachlor epoxide, malathion, methomyl, methoxychlor, mirex, nitrofen, toxaphene, tributyltin, triphenyltin, trifluralin, alkylphenol (C5-C9), nonylphenol, octylphenol, bisphenol A, di-2-ethylhexyl phthalate, butylbenzyl phthalate, di-n-butyl phthalate, dicyclohexyl phthalate, diethyl phthalate, benzo(a)pyrene, 2,4-dichlorophenol, di-2-ethylhexyl adipate, benzophenone, 4-nitrotoluene, aldicarb, benomyl, kepone (chlordecone), manzeb (mancozeb), maneb, metiram, metribuzin, cypermethrin, esfenvalerate, fenvalerate, permethrin, vinclozolin, zineb, ziram, dipentyl phthalate, dihexyl phthalate, and dipropyl phthalate.

Among the above-described environmental pollutants, most typical examples are dioxins and PCBs. Examples of the dioxins include 29 dioxins specified by Law Concerning Special Measures against Dioxins; i.e., dibenzodioxins such as 2,3,7,8-T₄CDD, 1,2,3,7,8-P₅CDD, 1,2,3,4,7,8-H₆CDD, 1,2,3,6,7,8-H₆CDD, 1,2,3,7,8,9-H₆CDD, 1,2,3,4,6,7,8-H₇CDD, and 1,2,3,4,6,7,8,9-O₈CDD; coplanar PCBs such as 3,4,4',5-T₄CB, 3,3',4,4'-T₄CB, 3,3',4,4',5-P₅CB, 3,3',4,4',5,5'-H₆CB, 2,3,3',4,4'-P₅CB, 2,3,4,4',5-P₅CB, 2,3',4,4',5-P₅CB, 2',3,4,4',5-P₅CB, 2,3,3',4,4',5-H₆CB, 2,3,3',4,4',5'-H₆CB, 2,3',4,4',5,5'-H₆CB, and 2,3,3',4,4',5,5'-H₇CB; and dibenzofurans such as 2,3,7,8-T₄CDF, 1,2,3,7,8-P₅CDF, 2,3,4,7,8-P₅CDF, 1,2,3,4,7,8-H₆CDF, 1,2,3,6,7,8-H₆CDF, 1,2,3,7,8,9-H₆CDF, 2,3,4,6,7,8-H₆CDF, 1,2,3,4,6,7,8-H₇CDF, 1,2,3,4,7,8,9-H₇CDF, and 1,2,3,4,6,7,8,9-O₈CDF.

Examples of the PCBs include 2-monochlorobiphenyl, 3-monochlorobiphenyl, and 4-monochlorobiphenyl;
2,2'-dichlorobiphenyl, 2,3-dichlorobiphenyl, 2,3'-dichlorobiphenyl, 2,4-dichlorobiphenyl, 2,4'-dichlorobiphenyl, 2,5-dichlorobiphenyl, 2,6-dichlorobiphenyl, 3,3'-dichlorobiphenyl, 3,4-dichlorobiphenyl, 3,4'-dichlorobiphenyl, 3,5-dichlorobiphenyl, and 4,4'-dichlorobiphenyl;
2,2',3-trichlorobiphenyl, 2,2',4-trichlorobiphenyl, 2,2',5-trichlorobiphenyl, 2,2',6-trichlorobiphenyl, 2,3,3'-trichlorobiphenyl, 2,3,4-trichlorobiphenyl, 2,3,4'-trichlorobiphenyl, 2,3,5-trichlorobiphenyl, 2,3,6-trichlorobiphenyl, 2,3',4-trichlorobiphenyl, 2,3',5-trichlorobiphenyl, 2,3',6-trichlorobiphenyl, 2,4,4'-trichlorobiphenyl, 2,4,5-trichlorobiphenyl, 2,4,6-trichlorobiphenyl, 2,4',5-trichlorobiphenyl, 2,4',6-trichlorobiphenyl, 2',3,4-trichlorobiphenyl, 2',3,5-trichlorobiphenyl, 3,3',4-trichlorobiphenyl, 3,3',5-trichlorobiphenyl, 3,4,4'-trichlorobiphenyl, 3,4,5-trichlorobiphenyl, and 3,4',5-trichlorobiphenyl;
2,2',3,3'-tetrachlorobiphenyl, 2,2',3,4-tetrachlorobiphenyl, 2,2',3,4'-tetrachlorobiphenyl, 2,2',3,5-tetrachlorobiphenyl, 2,2',3,5'-tetrachlorobiphenyl, 2,2',3,6-tetrachlorobiphenyl, 2,2',3,6'-tetrachlorobiphenyl, 2,2',4,4'-tetrachlorobiphenyl, 2,2',4,5-tetrachlorobiphenyl, 2,2',4,5'-tetrachlorobiphenyl, 2,2',4,6-tetrachlorobiphenyl, 2,2',4,6'-tetrachlorobiphenyl, 2,2',5,5'-tetrachlorobiphenyl, 2,2',5,6'-tetrachlorobiphenyl, 2,2',6,6'-tetrachlorobiphenyl, 2,3,3',4-tetrachlorobiphenyl, 2,3,3',4'-tetrachlorobiphenyl, 2,3,3',5-tetrachlorobiphenyl, 2,3,3',5'-tetrachlorobiphenyl, 2,3,3',6-tetrachlorobiphenyl, 2,3,4,4'-tetrachlorobiphenyl, 2,3,4,5-tetrachlorobiphenyl, 2,3,4,6-tetrachlorobiphenyl, 2,3,4',5-tetrachlorobiphenyl, 2,3,4',6-tetrachlorobiphenyl, 2,3,5,6-tetrachlorobiphenyl, 2,3',4,4'-tetrachlorobiphenyl, 2,3',4,5-tetrachlorobiphenyl, 2,3',4,5'-tetrachlorobiphenyl, 2,3',4,6-tetrachlorobiphenyl, 2,3',4',5-tetrachlorobiphenyl, 2,3',4',6-tetrachlorobiphenyl, 2,3',5,5'-tetrachlorobiphenyl, 2,3',5',6-tetrachlorobiphenyl, 2,4,4',5-tetrachlorobiphenyl, 2,4,4',6-tetrachlorobiphenyl, 2',3,4,5-tetrachlorobiphenyl, 3,3',4,4'-tetrachlorobiphenyl, 3,3',4,5-tetrachlorobiphenyl, 3,3',4,5'-tetrachlorobiphenyl, 3,3',5,5'-tetrachlorobiphenyl, and 3,4,4',5-tetrachlorobiphenyl;
2,2',3,3',4-pentachlorobiphenyl, 2,2',3,3',5-pentachlorobiphenyl, 2,2',3,3',6-pentachlorobiphenyl, 2,2',3,4,4'-pentachlorobiphenyl, 2,2',3,4,5-pentachlorobiphenyl, 2,2',3,4,5'-pentachlorobiphenyl, 2,2',3,4,6-pentachlorobiphenyl, 2,2',3,4,6'-pentachlorobiphenyl, 2,2',3,4',5-pentachlorobiphenyl, 2,2',3,4',6-pentachlorobiphenyl, 2,2',3,5,5'-pentachlorobiphenyl, 2,2',3,5,6-pentachlorobiphenyl, 2,2',3,5,6'-pentachlorobiphenyl, 2,2',3,5',6-pentachlorobiphenyl, 2,2',3,6,6'-pentachlorobiphenyl, 2,2',3',4,5-pentachlorobiphenyl, 2,2',3',4,6-pentachlorobiphenyl, 2,2',4,4',5-pentachlorobiphenyl, 2,2',4,4',6-pentachlorobiphenyl, 2,2',4,5,5'-pentachlorobiphenyl, 2,2',4,5,6'-pentachlorobiphenyl, 2,2',4,5',6-pentachlorobiphenyl, 2,2',4,6,6'-pentachlorobiphenyl, 2,3,3',4,4'-pentachlorobiphenyl, 2,3,3',4,5-pentachlorobiphenyl, 2,3,3',4',5-pentachlorobiphenyl, 2,3,3',4,5'-pentachlorobiphenyl, 2,3,3',4,6-pentachlorobiphenyl, 2,3,3',4',6-pentachlorobiphenyl, 2,3,3',5,5'-pentachlorobiphenyl, 2,3,3',5,6-pentachlorobiphenyl, 2,3,3',5',6-pentachlorobiphenyl, 2,3,4,4',5-pentachlorobiphenyl, 2,3,4,4',6-pentachlorobiphenyl, 2,3,4,5,6-pentachlorobiphenyl, 2,3,4',5,6-pentachlorobiphenyl, 2,3',4,4',5-pentachlorobiphenyl, 2,3',4,4',6-pentachlorobiphenyl, 2,3',4,5,5'-pentachlorobiphenyl, 2,3',4,5',6-pentachlorobiphenyl, 2',3,3',4,5-pentachlorobiphenyl, 2',3,4,4',5-pentachlorobiphenyl, 2',3,4,5,5'-pentachlorobiphenyl, 2',3,4,5,6'-pentachlorobiphenyl, 3,3',4,4',5-pentachlorobiphenyl, and 3,3',4,5,5'-pentachlorobiphenyl;
2,2',3,3',4,4'-hexachlorobiphenyl, 2,2',3,3',4,5-hexachlorobiphenyl, 2,2',3,3',4,5'-hexachlorobiphenyl, 2,2',3,3',4,6-hexachlorobiphenyl, 2,2',3,3',4,6'-hexachlorobiphenyl, 2,2',3,3',5,5'-hexachlorobiphenyl, 2,2',3,3',5,6-hexachlorobiphenyl, 2,2',3,3',5,6'-hexachlorobiphenyl, 2,2',3,3',6,6'-hexachlorobiphenyl, 2,2',3,4',4,5-hexachlorobiphenyl, 2,2',3,4,4',5'-hexachlorobiphenyl, 2,2',3,4,4',6-hexachlorobiphenyl, 2,2',3,4,4',6'-hexachlorabiphenyl, 2,2',3,4,4,5'-hexachlorobiphenyl, 2,2',3,4,5,6-hexachlorobiphenyl, 2,2',3,4,5,6'-hexachlorobiphenyl, 2,2',3,4,5',6-hexachlorobiphenyl, 2,2',3,4,6,6'-hexachlorobiphenyl, 2,2',3,4',5,5'-hexachlorobiphenyl, 2,2',3,4',5,6-hexachlorobiphenyl, 2,2',3,4',5,6'-hexachlorobiphenyl, 2,2',3,4',5',6-hexachlorobiphenyl, 2,2',3,4',6,6'-hexachlorobiphenyl, 2,2',3,5,5',6-hexachlorobiphenyl, 2,2',3,5,6,6'-hexachlorobiphenyl, 2,2',4,4',5,5'-hexachlorobiphenyl, 2,2',4,4',5,6'-hexachlorobiphenyl, 2,2',4,4',6,6'-hexachlorobiphenyl, 2,3,3',4,4',5-hexachlorobiphenyl, 2,3,3',4,4',5'-hexachlorobiphenyl, 2,3,3',4,4',6-hexachlorobiphenyl, 2,3,3',4,5,5'-hexachlorobiphenyl, 2,3,3',4,5,6-hexachlorobiphenyl, 2,3,3',4,5',6-hexachlorobiphenyl, 2,3,3',4',5,5'-hexachlorobiphenyl, 2,3,3`,4',5,6-hexachlorobiphenyl, 2,3,3',4',5',6-hexachlorobiphenyl, 2,3,3',5,5',6-hexachlorobiphenyl, 2,3,4,4',5,6-hexachlorobiphenyl, 2,3',4,4',5,5'-hexachlorobiphenyl, 2,3',4,4',5',6-hexachlorobiphenyl, and 3,3',4,4',5,5'-hexachlorobiphenyl;
2,2',3,3',4,4',5-heptachlorobiphenyl, 2,2',3,3',4,4',6-heptachlorobiphenyl, 2,2',3,3',4,5,5'-heptachlorobiphenyl, 2,2',3,3',4,5,6-heptachlorobiphenyl, 2,2',3,3',4,5,6'-heptachlorobiphenyl, 2,2',3,3',4,5',6-heptachlorobiphenyl, 2,2',3,3',4,6,6'-heptachlorobiphenyl, 2,2',3,3',4',5,6'-heptachlorobiphenyl, 2,2',3,3',5,5',6-heptachlorobiphenyl, 2,2',3,3',5,5,6'-heptachlorobiphenyl, 2,2',3,4,4',5,5'-heptachlorobiphenyl, 2,2',3,4,4',5,6-heptachlorobiphenyl, 2,2',3,4,4',5,6'-heptachlorobiphenyl, 2,2',3,4,4',5',6-heptachlorobiphenyl, 2,2',3,4,4',6,6'-heptachlorobiphenyl, 2,2',3,4,5,5',6-heptachlorobiphenyl, 2, 2',3,4,5,6,6'-heptachlorobiphenyl, 2,2',3,4',5,5',6-heptachlorobiphenyl, 2,2',3,4',5,6,6'-heptachlorobiphenyl, 2,2',3',4,4',5,5'-heptachlorobiphenyl, 2,2',3',4,4',5,6-heptachlorobiphenyl, 2,2',3',4,4',5',6-heptachlorobiphenyl, 2,2',3',4,5,5',6-heptachlorobiphenyl, and 2,2',3',4',5,5',6-heptachlorobiphenyl;
2,2',3,3',4,4',5,5'-octachlorobiphenyl, 2,2',3,3',4,4',5,6-octachlorobiphenyl, 2,2',3,3',4,4',5',6-octachlorobiphenyl, 2,2',3,3',4,4',6,6'-octachlorobiphenyl, 2,2',3,3',4,5,5',6-octachlorobiphenyl, 2,2',3,3',4,5,6,6'-octachlorobiphenyl, 2,2',3,3',4,5',6,6'-octachlorobiphenyl, 2,2',3,3',4',5,5',6-octachlorobiphenyl, 2,2',3,3',5,5',6,6'-octachlorobiphenyl, 2,2',3,4,4',5,5',6-octachlorobiphenyl, 2,2',3,4,4',5,6,6'-octachlorobiphenyl, and 2,3,3',4,4',5,5',6-octachlorobiphenyl;
2,2',3,3',4,4',5,5',6-nonachlorobiphenyl, 2,2',3,3',4,4',5,6,6'-nonachlorobiphenyl, and 2,2',3,3`,4,5,5',6,6'-nonachlorobiphenyl; and decachlorobiphenyl.

When an antibody to dioxins or PCBs, which is to be employed in the aforementioned present detection instrument, is prepared, a dioxin or PCB isomer (i.e., the target substance of the antibody) suitable for the case where toxicity of dioxins or PCBs contained in the test sample is estimated differs from that suitable for the case where the total amount of such environmental pollutants contained in the test sample is estimated. For example, in the case where the toxicity of dioxins contained in the test sample is estimated, 1,2,3,7,8-P₅CDD, 2,3,4,7,8-P₅CDF, 3,3',4,4',5-P₅CB, or the like, which greatly affects the total toxicity of dioxins contained in the test sample, is suitable for use as the target substance. Meanwhile, in the case where the total amount of dioxins contained in the test sample is estimated, 1,2,3,4,6,7,8,9-O₈CDD, 1,2,3,4,6,7,8,9-O₈CDF, 2,3',4,4',5-P₅CB, or the like, which is highly likely to be contained, as a dioxin, in a large amount in the test sample, is suitable for use as the target substance.

No particular limitations are imposed on the test sample, so long as it contains an environment-related low-molecular-weight substance such as a dioxin or PCB. Examples of the test sample include air samples, soil samples, and water samples such as lake water samples and sea water samples. Before being applied to the present detection instrument, the test sample may be subjected to any suitable preliminary treatment, such as dilution, filtration, or concentration, in accordance with its characteristics.

Two main modes of the present detection instrument are contemplated as described below.

A first mode of the present detection instrument comprises 1) a test sample application section with which a test sample is brought into contact; 2) a label product reaction section containing a label product containing, as a portion thereof, an antibody capable of binding to a target substance contained in the test sample, the label product being not bound to the reaction section; 3) an unbound label product capturing section containing an element capable of capturing the free label product (i.e., the label product which is not bound to the target substance), the element being bound to the capturing section; and 4) a detection section containing a detection element which, when coming into contact with the target substance bound to the label product, causes a visually observable change (hereinafter the first mode may be referred to as "the present detection instrument A").

In a second mode of the present detection instrument (low-molecular-weight substance detection instrument), a test sample is reacted with a labeled antibody containing, as a portion thereof, an antibody capable of binding to a target substance contained in the test sample, and the resultant reaction product is employed for detecting the target substance contained in the test sample (hereinafter the second mode may be referred to as "the present detection instrument B").

### Brief Description of the Drawings

Fig. 1 is a top plan view showing an embodiment of the present detection instrument A.
Fig. 2 is a vertical cross-sectional view showing the embodiment of the present detection instrument A.
Fig. 3 is a top plan view showing an embodiment of the present detection instrument B.
Fig. 4 is a vertical cross-sectional view showing the embodiment of the present detection instrument B.
Fig. 5 shows a labeled antibody sealed in a container, the antibody serving as a constitutive element of the present detection set.

### Best Mode for Carrying Out the Invention

### The present detection instrument A

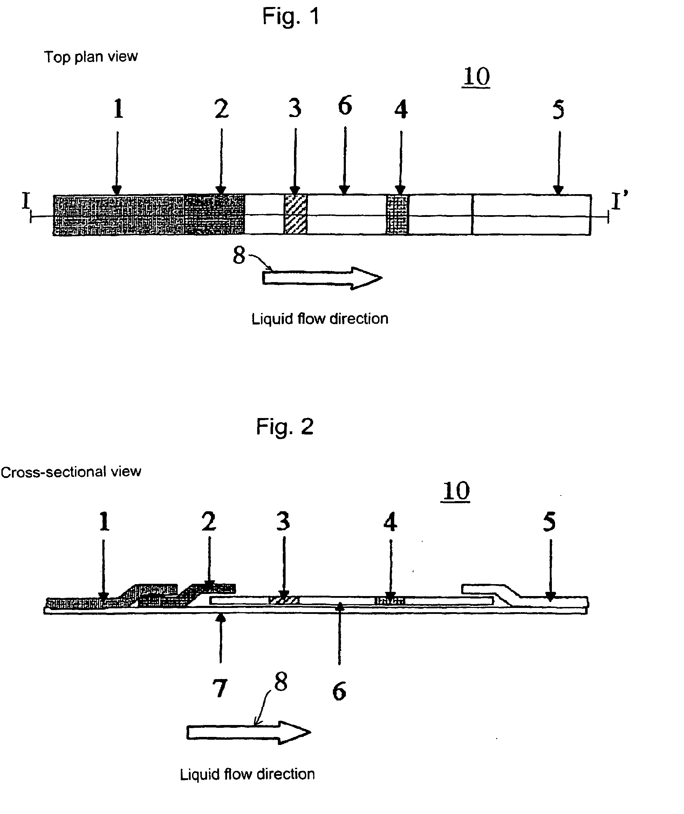

Fig. 1 is a top plan view showing an embodiment of the present detection instrument A; and Fig. 2 is a vertical cross-sectional view showing the present detection instrument A 10 of Fig. 1, as taken along solid line I-I'.

The present detection instrument A 10 includes a test sample application section 1, a label product reaction section 2, a membrane 6, an absorption section 5, and a strip-shaped thin film 7, wherein a portion of each of the sections 1, 2, and 5 and the entirety of the membrane 6 are bonded onto the upper surface of the thin film 7. The membrane 6 includes an unbound label product capturing section 3 and a detection section 4. More specifically, a first end portion of the test sample application section 1 is bonded to a first end portion of the thin film 7. A second end portion of the test sample application section 1 is in contact with the label product reaction section 2 so as to cover the upper surface of a first end portion of the section 2, the first end portion of the section 2 being located adjacent to the portion where the section 1 is bonded to the thin film 7 (i.e., the first end portion of the section 1) and being bonded to the thin film 7. A second end portion of the label product reaction section 2 is in contact with the membrane 6 so as to cover the upper surface of a first end portion of the membrane 6, the first end portion of the membrane 6 being located adjacent to the portion where the section 2 is bonded to the thin film 7 (i.e., the first end portion of the section 2) and the entire surface of the membrane 6 being bonded to the thin film 7. A first end portion of the absorption section 5 is bonded to a second end portion of the thin film 7. A second end portion of the absorption section 5 is in contact with the membrane 6 so as to cover the upper surface of a second end portion of the membrane 6.

The unbound label product capturing section 3 and the detection section 4 are provided on the membrane 6 by fixating chemical components constituting these sections onto the membrane 6.

In the present detection instrument A 10, a test sample is brought into contact with the test sample application section 1, and the test sample migrates from the section 1 toward the absorption section 5 by means of capillary action, whereby a low-molecular-weight substance contained in the test sample is detected (hereinafter, the side of the present detection instrument A on which the test sample application section is provided may be referred to as "the upstream side," and the side of the instrument A on which the absorption section is provided may be referred to as "the downstream side"). In Figs. 1 and 2, an arrow 8 shows the direction of migration of the test sample from the upstream side to the downstream side.

No particular limitations are imposed on the material of the test sample application section 1, so long as the test sample brought into contact with the section 1 can migrate to the label product reaction section 2, which is located downstream of the section 1, by means of capillary action. Examples of the material include papers such as filter paper; fabrics such as raised fabric; cotton; and glass fiber.

When the test sample comes into contact with the test sample application section 1 of the present detection instrument A 10, and then reaches the portion of the section 1 that is in contact with the label product reaction section 2, the test sample migrates to the section 2 by means of capillary action.

The label product reaction section 2 contains, in an unbound state, a label product containing, as a portion thereof, an antibody capable of binding to a target substance contained in the test sample.

The antibody employed in the label product reaction section 2 can be produced through a customary method by use of an environmental pollutant to be detected, serving as an immunogen (if desired, the immunogen may be formed of a carrier protein and a hapten which targets the environmental pollutant to be detected).

In the case where the antibody employed in the section 2 is a polyclonal antibody, the antibody can be produced from immune serum derived from an animal immunized with an environmental pollutant (e.g., a dioxin or a PCB) to be detected, serving as an immunogen. Meanwhile, in the case where the antibody employed in the section 2 is a monoclonal antibody, the antibody can be produced through the following procedure: a hybridoma is prepared from myeloma cells of an animal and immunocytes of an animal which is immunized in a manner similar to that of the aforementioned polyclonal antibody, and a clone which produces an antibody recognizing an environmental pollutant (e.g., a dioxin or a PCB) to be detected is selected by use of the hybridoma, followed by culturing of the clone.

No particular limitations are imposed on the animal to be immunized, and the animal may be, for example, mouse or rat. In the case where the monoclonal antibody is produced, preferably, the animal is chosen in consideration of compatibility with myeloma cells employed for cell fusion.

Immunization can be performed by means of a generally employed method; for example, a method in which the aforementioned immunogen is administered to an animal to be immunized through, for example, intravenous, intradermal, subcutaneous, or intraperitoneal injection.

More specifically, the aforementioned immunogen―which is, if desired, employed in combination with a typical adjuvant―can be administered several times to an animal to be immunized every two to four weeks through the aforementioned means, to thereby prepare immune serum for producing the polyclonal antibody, or immunocytes (e.g., immunized spleen cells) for producing the monoclonal antibody.

In the case where the monoclonal antibody is produced, the immunocytes may be subjected to cell fusion with known myeloma cells serving as parental cells. Examples of the known myeloma cells include SP2/0-Ag14, P3-NS1-1-Ag4-1, MPC11-45, and 6.TG1.7 (which are derived from mouse); 210.RCY.Agl.2.3 (which is derived from rat); and SKO-007 and GM15006TG-A12 (which are derived from human).

Cell fusion between the aforementioned immunocytes and myeloma cells can be carried out by means of a generally known method, such as the method of Köhler and Milstein (Köhler, G. and Milstein, C., Nature, 256, 495 (1975)).

More specifically, the cell fusion is carried out in a generally employed culture medium to which, if desired, an adjuvant (e.g., dimethyl sulfoxide) is added for enhancing fusion efficiency, in the presence of a generally known fusion promoter such as polyethylene glycol (PEG) or Sendai virus (HVJ), to thereby prepare a hybridoma.

Separation of a target hybridoma can be performed through culturing in a generally employed screening medium such as HAT (hypoxanthine, aminopterin, and thymidine) medium. Specifically, a target hybridoma can be separated by performing culturing in the screening medium for a sufficient time for apoptosis of cells other than the hybridoma. Through a typical limiting dilution technique, the thus-prepared hybridoma can be employed for screening and monocloning of a target monoclonal antibody.

A cell strain which produces the target monoclonal antibody can be screened by means of a generally employed screening method, such as ELISA, the plaque method, spotting, agglutination reaction, the Ouchterlony method, or RIA.

The thus-prepared hybridoma which produces the monoclonal antibody recognizing an environmental pollutant can be subcultured in a typical medium and stored in liquid nitrogen for a long period of time.

The target monoclonal antibody can be collected from the hybridoma by means of, for example, a method in which the hybridoma is cultured in a conventional technique, and the monoclonal antibody is obtained from the resultant supernatant; or a method in which the hybridoma is administered to an animal exhibiting compatibility with the hybridoma to thereby proliferate the hybridoma, and the monoclonal antibody is obtained from ascites of the animal.

The above-obtained polyclonal or monoclonal antibody may be purified through generally employed means, such as salting out, gel filtration, or affinity chromatography.

In general, the label product employed in the label product reaction section 2 is preferably a labeled antibody prepared by labeling the aforementioned antibody to an environmental pollutant with a labeling substance.

Examples of the labeling substance which may be employed include metallic colloidal particles such as gold colloidal particles; latex particles; and enzymes exhibiting color-developing ability, such as horseradish peroxidase. Such a labeling substance and the antibody are bound together by means of a customary method, and the thus-bound product is employed in the label product reaction section 2.

The aforementioned label product must be present, in an unbound state, in the material constituting the label product reaction section 2, such that, regardless of whether or not a target substance is present in the test sample, the label product can migrate, together with the test sample which has migrated from the test sample application section 1, toward the unbound label product capturing section 3, which is located downstream of the section 2, by means of capillary action. Therefore, preferably, the aforementioned label product is simply held in the label product reaction section through, for example, permeation of the label product into the material of the reaction section. The material of the label product reaction section 2 is preferably a material which facilitates such simple holding or elution of the label product. Specific examples of such a preferred material include papers such as filter paper; fabrics such as raised fabric; cotton; and glass fiber.

When a target substance is present in the test sample, a labeled complex formed through binding between the target substance and the label product, as well as the free label product (i.e., the label product not bound to the target substance) migrate toward the downstream side, whereas when a target substance is not present in the test sample, merely the free label product migrates toward the downstream side.

In the present detection instrument A 10, the unbound label product capturing section 3 and the detection section 4 are provided on the membrane, the section 4 being located downstream of the section 3. The membrane is preferably formed of a material which enables a liquid phase to migrate therethrough by means of capillary action, such as a porous chromatomembrane or a nitrocellulose membrane. Particularly, a porous chromatomembrane is preferred, since a labeling substance, such as a protein (e.g., horseradish peroxidase), metallic colloidal particles (e.g., gold colloidal particles), or latex particles, can readily migrate through the membrane.

The labeled complex and/or the free label product, which has migrated from the upstream side by means of capillary action, comes into contact with the unbound label product capturing section 3. The unbound label product capturing section has an element capable of capturing merely the free label product (i.e., the label product not bound to the target substance). Examples of the element include a substance which can react with and bind to the antibody to the target substance, the antibody constituting the label product. Specific examples include the target substance per se, and substances which are similar to the target substance and can react with and bind to the antibody. The target substance or the like must be bound to the unbound label product capturing section 3, such that the target substance or the like does not migrate as a liquid phase migrates through the section 3. Since the target substance or the like has low molecular weight, preferably, the target substance or the like is bound to a carrier protein such as BSA, and the target substance or the like bound to the protein is bound and fixated to the unbound label product capturing section 3 by means of a customary protein fixation method.

As described above, the unbound label product capturing section 3 has an element capable of capturing merely the free label product (i.e., the label product not bound to the target substance). Therefore, in the case where the test sample contains a target substance, when the labeled complex and the free label product migrate from the upstream side to the unbound label product capturing section 3, merely the free label product is captured by the section 3, and merely the labeled complex migrates toward the downstream side. Meanwhile, in the case where the test sample contains no target substance, merely the free label product migrates to the unbound label product capturing section 3, the free label product is completely captured by the section 3, and merely a liquid phase containing no label product migrates to the downstream side.

Subsequently, the liquid phase which has passed through the unbound label product capturing section 3 comes into contact with the detection section 4, which is provided downstream of the section 3. The detection section 4 has a labeling substance visualization element capable of visualizing the labeling substance employed in the label product. The labeling substance visualization element corresponds to the employed labeling substance. When, for example, the label product employs an originally visually observable labeling substance (e.g., gold colloidal particles), the visualization element is preferably a detection element capable of capturing the labeled complex in a concentrated form; for example, an antibody specific to the antibody specific to the employed environmental pollutant (i.e., an anti-immunoglobulin antibody). The anti-immunoglobulin antibody is preferably an antibody which binds to a portion (other than the paratope) of the antibody specific to the employed environmental pollutant, such as an anti-Fc antibody.

When such an anti-immunoglobulin antibody is densely fixated, as a detection element, to the detection section 4, the labeling substance which has migrated together with a liquid phase can be concentrated and visualized by the section 4.

Meanwhile, in the case where the employed labeling substance is an enzyme having a color-developing ability, such as horseradish peroxidase; i.e., in the case where a substrate must be brought into contact with the enzyme at the detection section 4 for color development, preferably, the detection element of the detection section 4 is prepared by binding an anti-immunoglobulin antibody to, for example, a reagent capable of visualizing the employed labeling substance, rather than an anti-immunoglobulin antibody being employed solely as the detection element.

In any of the aforementioned cases, in order to visualize the labeling substance, the detection element such as an anti-immunoglobulin antibody is preferably bound to the detection section 4, such that the detection element does not migrate from the section 4 together with a liquid phase by means of capillary action. No particular limitations are imposed on the binding state of the detection element, so long as the detection element does not migrate from the detection section 4 together with a liquid phase. For example, in the case where the detection element is an antibody which binds to a portion (other than the paratope) of the antibody specific to an environmental pollutant to be detected, when a solution of the detection element (antibody) is added dropwise to the corresponding portion of the detection section 4, a desired binding state of the detection element is established.

Finally, the absorption section 5 absorbs a liquid phase which has migrated from the upstream side toward the downstream side by means of capillary action. In the present detection instrument A, the absorption section 5 is an optional element. However, the absorption section 5 is preferably provided on the detection instrument for facilitating migration of the test sample through the membrane.

As described above, in the case where the test sample contains a target substance, in the present detection instrument A 10, the target substance (i.e., low-molecular-weight compound) binds, at the label product reaction section 2, to a label product which reacts specifically with the target substance, to thereby form a labeled complex. Subsequently, the thus-formed labeled complex migrates through the membrane 6. The labeled complex is not captured by the unbound label product capturing section 3, but the complex reaches the detection section 4. Since a substance capable of visualizing the labeled complex is fixated onto the detection section 4, the complex is concentrated at the section 4, whereby a visually observable band of the complex is detected.

Meanwhile, in the case where the test sample contains no target substance, a labeled complex as described above is not formed at the label product reaction section 2, and the free label product migrates through the membrane together with a liquid phase. The free label product is completely captured by the unbound label product capturing section 3, and thus no label product reaches the detection section 4. As a result, a band of the label product is not detected at the detection section 4.

As described above, the present invention provides means for detecting, by use of a positive index, an environmental pollutant, which has conventionally been difficult to detect.

### The present detection instrument B

Although the present detection instrument A described above is considered to be very beneficial to the industry, the instrument A tends not to exhibit high detection sensitivity as expected.

Specifically, the present detection instrument A tends to raise, for example, the following problems.
1) When a test sample is brought into contact with the test sample application section, a solvent contained in the test sample and the label product of the label product reaction section rapidly reach the unbound label product capturing section provided on the membrane. Therefore, when the label product reaction section contains a large amount of the label product, the capturing section encounters difficulty in completely capturing the unbound label product. As a result, false-positive reaction may occur at the detection section, even when the test sample contains no target substance.
2) When treatment with a surfactant is carried out for promoting migration of the label product from the label product reaction section to the membrane, antigen-antibody reaction which is supposed to occur is inhibited. Therefore, even when the test sample contains no target substance, the capturing section may fail to completely capture the unbound label product, leading to occurrence of false-positive reaction at the detection section.
3) When the test sample is brought into contact with the test sample application section, a solvent contained in the test sample and the label product of the label product reaction section rapidly migrate through the membrane, and thus only a portion of the target substance contained in the test sample reacts with the label product. Therefore, even when the amount of the test sample is increased, difficulty would be encountered in enhancing detection sensitivity.

While avoiding the problems associated with the present detection instrument A, the present detection instrument B can detect the target substance contained in the test sample; i.e., a low-molecular-weight substance such as an environmental pollutant (e.g., a dioxin).

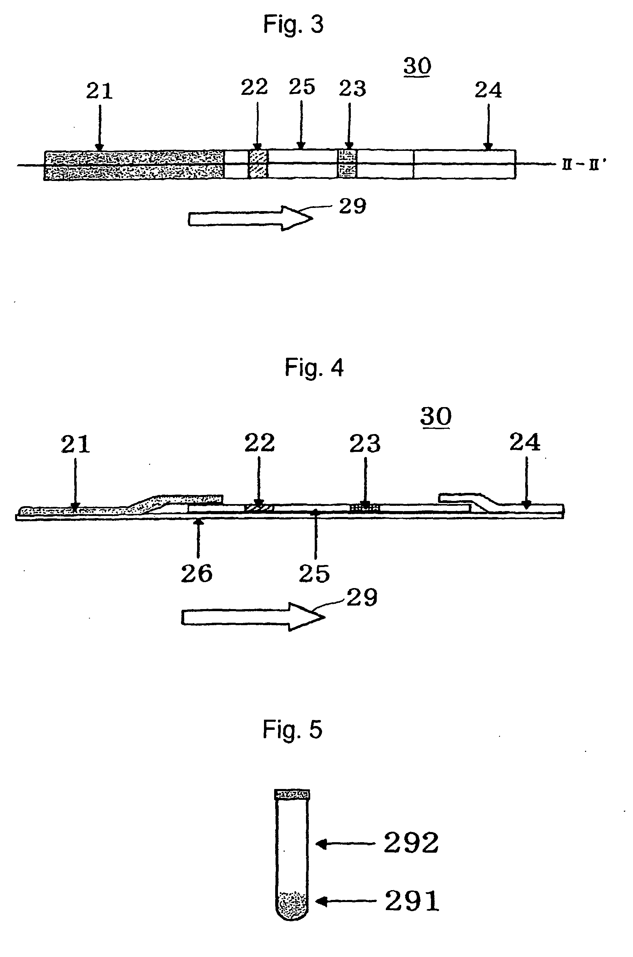

Fig. 3 is a top plan view showing an embodiment of the present detection instrument B; and Fig. 4 is a vertical cross-sectional view showing the present detection instrument B 30 of Fig. 3, as taken along solid line II-II'.

The present detection instrument B 30 includes a reaction product contact section 21, a membrane 25, an absorption section 24, and a strip-shaped thin film 26, wherein portions of the respective sections 21 and 24 and the entirety of the membrane 25 are bonded onto the upper surface of the thin film. The membrane 25 includes an unbound labeled antibody capturing section 22 and a detection section 23.

More specifically, a first end portion of the reaction product contact section 21 is bonded to a first end portion of the thin film 26. A second end portion of the reaction product contact section 21 is in contact with the membrane 25 so as to cover the upper surface of a first end portion of the membrane 25, the first end portion of the membrane 25 being located adjacent to the portion where the section 21 is bonded to the thin film 26 (i.e., the first end portion of the section 21) and the entire surface of the membrane 25 being bonded to the thin film 26. A first end portion of the absorption section 24 is bonded to a second end portion of the thin film 26. A second end portion of the absorption section 24 is in contact with the membrane 25 so as to cover the upper surface of a second end portion of the membrane 25.

The unbound labeled antibody capturing section 22 and the detection section 23 are provided on the membrane 25 by fixating chemical components constituting these sections onto the membrane 25.

The present detection instrument B 30 employs immunochromatography, in which a reaction product which has previously formed through contact between a test sample and a labeled antibody is brought into contact with the reaction product contact section 21, rather than the test sample per se being brought into contact with, for example, a test sample application section. Therefore, the present detection instrument B 30 differs from a conventional detection instrument employing immunochromatography in which a test sample *per se* is brought into contact with a test sample application section.

In the case of the present detection instrument B 30, the test sample can be sufficiently reacted with the labeled antibody. Therefore, when a reaction product formed through this reaction is applied to the present detection instrument B 30, the labeled antibody can be prevented from rapidly reaching the unbound labeled antibody capturing section, whereby the aforementioned false-positive reaction is suppressed, and detection sensitivity is enhanced. Thus, the detection instrument can conveniently and sensitively detect, for example, a low-molecular-weight substance present in the environment which, even in a trace amount, would cause problems.

In the present detection instrument B 30, the above-formed reaction product is brought into contact with the reaction product contact section 21, and the reaction product migrates from the section 21 toward the absorption section 24 by means of capillary action, whereby a low-molecular-weight substance contained in the test sample is detected (hereinafter, the side of the present detection instrument B on which the reaction product contact section is provided may be referred to as "the upstream side," and the side of the instrument B on which the absorption section is provided may be referred to as "the downstream side"). In Figs. 3 and 4, an arrow 29 shows the direction of migration of the reaction product from the upstream side to the downstream side.

Since the labeled antibody is reacted with the test sample before the reaction product is applied to the present detection instrument B, the labeled antibody is not an essential element of the present detection instrument B. However, the labeled antibody is essential for detecting a low-molecular-weight substance by use of the present detection instrument B.

The present invention provides a method of using the present detection instrument B, which comprises bringing, into contact with the reaction product contact section, a reaction product formed from a test sample and a labeled antibody capable of binding to a target substance contained in the test sample; and detecting a complex of the labeled antibody and the target substance, and/or the free labeled antibody (i.e., the labeled antibody which is not bound to the target substance), the complex and the free labeled antibody being contained in the reaction product, to thereby detect the target substance contained in the test sample (hereinafter the method may be referred to as "the present use method").

The present invention also provides a set for detecting a low-molecular-weight substance, comprising the present detection instrument B and a labeled antibody as constitutive elements for performing the present use method (hereinafter the set may be referred to as "the present detection set").

The antibody which can be employed as a portion of the labeled antibody employed as an element of the present detection set; i.e., an antibody capable of binding to a target substance contained in the test sample, may be a polyclonal antibody or a monoclonal antibody. Such an antibody can be produced through a customary method by use, as an immunogen, of a low-molecular-weight substance to be detected (e.g., an environmental pollutant) (if desired, the immunogen may be formed by binding a carrier protein to a hapten which targets an environmental pollutant to be detected). This customary production method is similar to that described above in the case of the present detection instrument A.

In the case where the present detection instrument B is employed, the labeled antibody is preferably maintained in a dry condition. For example, a labeled antibody 291 is dried by means of, for example, thermal drying, vacuum drying, or freeze-drying, in the presence of a stabilizer such as a sugar, a surfactant, or a polyhydric alcohol (e.g., glycerol), and the thus-dried labeled antibody 291 is stored in, for example, a sealable container 292 while maintaining the antibody in a dry condition (preferably using a desiccant) (Fig. 5: A typical embodiment of the present detection set includes the present detection instrument B 30, and the labeled antibody 291 sealed in the container 292.).

In the case where a test sample is subjected to detection by means of the present use method by use of the present detection instrument B 30 or the present detection set including the instrument as a constitutive element, when a target substance is present in the test sample, a labeled complex formed through binding between the target substance and the labeled antibody, and the free labeled antibody (i.e., the labeled antibody not bound to the target substance), which are contained in the reaction product, migrate toward the downstream side. Meanwhile, when a target substance is not present in the test sample, since a labeled complex is not contained in the reaction product, merely the free labeled antibody migrates toward the downstream side.

As described above, in the present detection instrument B 30, the unbound labeled antibody capturing section 22 and the detection section 23 are provided on the membrane 25, the section 23 being located downstream of the section 22. The membrane is preferably formed of a material which enables a liquid phase to migrate therethrough by means of capillary action, such as a porous chromatomembrane or a nitrocellulose membrane. Particularly, a porous chromatomembrane is preferred, since a labeling substance, such as a protein (e.g., horseradish peroxidase), metallic colloidal particles (e.g., gold colloidal particles), or latex particles, can readily migrate through the membrane.

Firstly, the labeled complex and/or the free labeled antibody, which has migrated from the upstream side by means of capillary action, comes into contact with the unbound labeled antibody capturing section 22. The unbound labeled antibody capturing section has an element capable of capturing merely the free labeled antibody (i.e., the labeled antibody not bound to the target substance). Examples of the element include a substance which can react with and bind to the antibody to the target substance, the antibody constituting the labeled antibody. Specific examples include the target substance *per se,* and substances which are similar to the target substance and can react with and bind to the antibody. The target substance or the like must be bound to the unbound labeled antibody capturing section 22, such that the target substance or the like does not migrate as a liquid phase migrates through the section 22. Since the target substance or the like has low molecular weight, preferably, the target substance or the like is bound to a carrier protein such as BSA, and the target substance or the like bound to the protein is bound and fixated to the unbound labeled antibody capturing section 22 by means of a customary protein fixation method.

As described above, the unbound labeled antibody capturing section 22 has an element capable of capturing merely the free labeled antibody (i.e., the labeled antibody not bound to the target substance). Therefore, in the case where the test sample contains a target substance, when the labeled complex and the free labeled antibody migrate from the upstream side to the unbound labeled antibody capturing section 22, merely the free labeled antibody is captured by the section 22, and merely the labeled complex migrates toward the downstream side. Meanwhile, in the case where the test sample contains no target substance, merely the free labeled antibody migrates to the unbound labeled antibody capturing section 22. Therefore, the free labeled antibody is almost completely captured by the section 22, and a liquid phase containing virtually no labeled antibody migrates to the downstream side.

Subsequently, the liquid phase which has passed through the unbound labeled antibody capturing section 22 comes into contact with the detection section 23, which is provided downstream of the section 22. The detection section 23 has a labeling substance visualization element for visualizing the labeling substance employed in the labeled antibody. The labeling substance visualization element corresponds to the employed labeling substance. When, for example, the labeled antibody employs an originally visually observable labeling substance (e.g., gold colloidal particles), the visualization element is preferably a detection element capable of capturing the labeled complex in a concentrated form; for example, an antibody specific to the antibody specific to the employed environmental pollutant (i.e., an anti-immunoglobulin antibody). The anti-immunoglobulin antibody is preferably an antibody which binds to a portion (other than the paratope) of the antibody specific to the employed environmental pollutant, such as an anti-Fc antibody.

When such an anti-immunoglobulin antibody is densely fixated, as a detection element, to the detection section 23, the labeling substance which has migrated together with a liquid phase can be concentrated and visualized by the section 23.

Meanwhile, in the case where the employed labeling substance is an enzyme having a color-developing ability, such as horseradish peroxidase; i.e., in the case where a substrate must be brought into contact with the enzyme at the detection section 23 for color development, preferably, the detection element of the detection section 23 is prepared by binding an anti-immunoglobulin antibody to, for example, a reagent capable of visualizing the employed labeling substance, rather than an anti-immunoglobulin antibody being employed solely as the detection element.

In any of the aforementioned cases, in order to visualize the labeling substance, the detection element such as an anti-immunoglobulin antibody is preferably bound to the detection section 23, such that the detection element does not migrate from the section 23 together with a liquid phase by means of capillary action. No particular limitations are imposed on the binding state of the detection element, so long as the detection element does not migrate from the detection section 23 together with a liquid phase. For example, in the case where the detection element is an antibody which binds to a portion (other than the paratope) of the antibody specific to an environmental pollutant to be detected, when a solution of the detection element (antibody) is added dropwise to the corresponding portion of the detection section 23, a desired binding state of the detection element is established.

Finally, the absorption section 24 absorbs a liquid phase which has migrated from the upstream side toward the downstream side. In the present detection instrument B 30, the absorption section 24 is an optional element. However, the absorption section 24 is preferably provided on the detection instrument for facilitating migration of the test sample through the membrane.

As described above, in the case where the test sample contains a target substance, in the present detection instrument B 30, the target substance (i.e., low-molecular-weight compound) binds, in the container 292, to the labeled antibody 291 which reacts specifically with the target substance, to thereby form a labeled complex. Subsequently, the thus-formed labeled complex comes into contact with the reaction product contact section 21, and then migrates through the membrane 25. The labeled complex is not captured by the unbound labeled antibody capturing section 22, but the complex reaches the detection section 23. Since a substance capable of visualizing the labeled complex is fixated onto the detection section 23, the complex is concentrated at the section 23, whereby a visually observable band of the complex is detected.

Meanwhile, in the case where the test sample contains no target substance, a labeled complex as described above is not formed, and the free labeled antibody migrates through the membrane together with a liquid phase. The free labeled antibody is almost completely captured by the unbound labeled antibody capturing section 22, and thus virtually no labeled antibody reaches the detection section 23. As a result, a band of the labeled antibody is virtually not detected at the detection section 23.

Thus, when the present use method is carried out by use of the present detection instrument B 30 or the present detection set including the instrument as an element, an environmental pollutant, etc. can be detected by use of a positive index. Furthermore, as described above, there can be suppressed false-positive reaction due to the presence of an excess amount of unbound labeled antibody, which reaction tends to occur in the aforementioned conventional detection instrument employing immunochromatography.

The present detection instrument B 30 is an embodiment of the present invention, and different embodiments of the invention may be made within the scope of the invention. For example, the present invention may be applied to immunochromatography, in which a labeled antibody that is not captured by a line bound onto a membrane, which line can capture a reaction product (labeled complex), is detected, whereby a target substance is detected by use of a negative index.

### Examples

The present invention will next be described in more detail by way of Examples.

### A. Example in relation to the present detection instrument A

### A1. Formation of immunogen for preparation of Co-PCB #126 monoclonal antibody

A hapten targeting Co-PCB #126; i.e., 6-[(3,3,4,5-tetrachlorobiphenyl-4-yl)oxy]hexanoic acid (low-molecular-weight compound 126), was synthesized by means of a slight modification of the 6-[(3,3,4-trichlorobiphenyl-4-yl)oxy]hexanoic acid synthesis method which had previously been reported by Ya-Wen Chiu, *et al.* (Analytical Chemistry, 1995, 67, 3829).

Specifically, 2-chloroanisole and 3,4,5-trichloroaniline were subjected to cadogan coupling reaction, to thereby synthesize 3,3',4',5'-tetrachloro-4-methoxybiphenyl (yield: 12%); the thus-synthesized 3,3',4',5'-tetrachloro-4-methoxybiphenyl was subjected to demethylation reaction with boron tribromide, to thereby form 3,3',4',5'-tetrachloro-4-hydroxybiphenyl (yield: 90%); the thus-formed 3,3',4',5'-tetrachloro-4-hydroxybiphenyl was reacted with ethyl 6-bromohexanoate, to thereby form 6-[(3,3',4',5'-tetrachloro-4-yl)oxy] hexanoate (yield: 90%); and the thus-formed [(3,3',4',5'-tetrachloro-4-yl)oxy] hexanoate was hydrolyzed with an alkali, to thereby synthesize 6-[(3,3',4',5'-tetrachloro-4-yl)oxy]hexanoic acid (yield: 95%).

The thus-synthesized hapten was bound to limulus hemocyanin (KLH) by means of the NHS ester method employing N-hydroxysuccinimide (P. Schneider and B. D. Hammock, Journal of Agricultural and Food Chemistry, 1992, 2, 85), and the resultant product was employed as an antigen for preparation of a Co-PCB #126 monoclonal antibody.

Specifically, the above-synthesized hapten was formed into an active ester by use of 1-ethyl-3-[3-(dimethylamino)propyl]carbodiimide and N-hydroxysuccinimide, and then reacted with KLH to form an amide bond between the hapten and the amino group of KLH, thereby yielding a hapten-KLH conjugate.

### A2. Preparation of Co-PCB #126 monoclonal antibody

A balb/c mouse was immunized with the Co-PCB #126 low-molecular-weight compound-KLH conjugate which had been formed and purified through the above-described method and with Ribi Adjuvant System (product of Corixa) at intervals of two weeks (up to eight times), and then subjected to booster at the caudal vein. Thereafter, antibody-producing cells in the spleen and myeloma cells were subjected to cell fusion by means of a customary method. The resultant culture supernatant containing hybridomas was subjected to screening by use of a plate on which the Co-PCB #126 low-molecular-weight compound-protein conjugate was immobilized, to thereby select a hybridoma which produces an antibody that reacts specifically with Co-PCB #126.

The antibody employed in the present experiment was prepared through the following procedure: the above-selected hybridoma was intraperitoneally administered to a mouse treated with pristane, ascites was collected from the mouse by means of a customary method, and then the thus-collected ascites was subjected to purification through anion-exchange chromatography.

### A3. Preparation of low-molecular-weight compound-protein conjugate to be immobilized onto unbound label product caputuring section of chromatomembrane

The low-molecular-weight compound 126 which had been synthesized above in A1 was bound to bovine serum albumin (BSA) in a manner similar to that described above in A1, and the resultant product was purified through dialysis. The thus-obtained product was employed as a low-molecular-weight compound-protein conjugate to be immobilized onto an unbound label product capturing section of a chromatomembrane.

### A4. Preparation of chromatomembrane

The low-molecular-weight compound 126-BSA conjugate which had been formed above in A3, which constitutes an unbound label product capturing section, was applied to Hi-Flow Plus Membrane (HF90, 25 mm × 30 cm, Millipore) at a position 5 mm away from the lower end thereof. Meanwhile, anti-mouse IgG rabbit antibody (Zymed), which constitutes a detection section, was applied to the membrane at a position 15 mm away from the lower end thereof. The solutions of the conjugate and antibody were applied, by use of XYZ Handling System (BioDot), to the membrane so as to form lines. After completion of application, the membrane was allowed to stand at room temperature overnight for drying, and then was employed as a chromatomembrane.

### A5. Preparation of gold-colloidal-particles-labeled anti-Co-PCB monoclonal antibody

Gold colloidal particles (average particle size: about 40 nm, BBInternational) were bound to an anti-Co-PCB monoclonal antibody according to the protocol by BBInternational. The OD520 of the resultant gold-colloid-labeled anti-Co-PCB monoclonal antibody solution was regulated to 5 to 6, and the solution was refrigerated until employed.

### A6. Preparation of gold colloid pad (label product reaction section)

A glass fiber-made conjugate pad (10 mm × 30 cm, product of Millipore) was impregnated with the above-prepared gold-colloid-labeled antibody solution (final concentration: 16%), and then completely dried by use of a vacuum drying machine. The resultant pad was employed as a gold colloid pad.

### A7. Preparation of sample pad (test sample application section)

A sample pad (20 mm × 30 cm, Millipore) was impregnated with a PBS solution containing 2% sucrose, 1% BSA, and 1% Triton X-100, and then completely dried by use of a vacuum drying machine. The resultant pad was employed as a sample pad.

### A8. Assembly of the present detection instrument A

The above-prepared sample pad, gold colloid pad, and chromatomembrane were mounted on a laminate card (Millipore) as shown in Figs. 1 and 2. Furthermore, an absorption pad (17 mm × 30 cm, product of Millipore) was mounted on the card, and subsequently, the resultant card was cut into pieces having a width of 5 mm by use of a guillotine cutter (BioDot). Each of the pieces was employed as the present detection instrument A.

### A9. Measurement of Co-PCB #126

Co-PCB #126 was diluted with a TBS solution containing 25% DMSO so that the Co-PCB #126 concentration became 10 ppm, 5 ppm, 2.5 ppm, 1.25 ppm, 1 ppm, or 0.1 ppm. The thus-diluted Co-PCB #126 sample (150 µL) was added dropwise to the test sample application section of the present detection instrument assembled above in A8. The degree of coloring of the detection section was evaluated through visual observation at different points in time (see Table 1) after addition of the sample. The results are shown in Table 1.

**Table 1**

| Co-PCB 126 concentration (ppm) | Degree of coloring of detection section | | | |
|---|---|---|---|---|
| | 3 minutes later | 5 minutes later | 10 minutes later | 20 minutes later |
| 10 | - | - | + | ++ |
| 5 | - | + | ++ | ++ |
| 2.5 | + | + | ++ | ++ |
| 1.25 | + | + | ++ | ++ |
| 1 | - | - | + | + |
| 0.1 | - | - | - | - |
| 0 | - | - | - | - |
| [Evaluation criteria] ++: high coloring degree, +: low coloring degree, -: no coloring | | | | |

### A10. Results

As is clear from Table 1, when the concentration of Co-PCB in the sample is 1 ppm or more, the sample is determined to be positive through visual evaluation 10 minutes after addition of the sample to the detection instrument.

### B. Example in relation to the present detection instrument B

### B1. Formation of immunogen for preparation of Co-PCB #118 monoclonal antibody

A low-molecular-weight compound targeting Co-PCB #118; i.e., 6-[(3,2',4',5'-tetrachlorobiphenyl-4-yl)oxy]hexanoic acid (low-molecular-weight compound 118), was synthesized by means of a slight modification of the 6-[(3,3', 4'-trichlorobiphenyl-4-yl)oxy]hexanoic acid synthesis method which had previously been reported by Ya-Wen Chiu, *et al*. (Analytical Chemistry, 1995, 67, 3829).

The thus-synthesized low-molecular-weight compound was bound to limulus hemocyanin (KLH) by means of the NHS ester method employing N-hydroxysuccinimide (P. Schneider and B. D. Hammock, Journal of Agricultural and Food Chemistry, 1992, 2, 85), and the resultant product was employed as an antigen for preparation of a Co-PCB #118 monoclonal antibody.

### B2. Preparation of Co-PCB #118 monoclonal antibody

A balb/c mouse was immunized with the Co-PCB #118 low-molecular-weight compound-KLH conjugate which had been formed and purified through the above-described method and with Ribi Adjuvant System (product of Corixa) at intervals of two weeks (up to eight times), and then subjected to booster at the caudal vein. Thereafter, antibody-producing cells in the spleen and myeloma cells were subjected to cell fusion by means of a customary method. The resultant culture supernatant containing hybridomas was subjected to screening by use of a plate on which the Co-PCB #118 low-molecular-weight compound-protein conjugate was immobilized, to thereby select a hybridoma which produces an antibody that reacts specifically with Co-PCB #118.

The antibody employed in the present experiment was prepared through the following procedure: the above-selected hybridoma was intraperitoneally administered to a mouse treated with pristane, ascites was collected from the mouse by means of a customary method, and then the thus-collected ascites was subjected to purification through anion-exchange chromatography.

### B3. Preparation of low-molecular-weight compound-protein conjugate to be immobilized onto unbound labeled antibody caputuring section of chromatomembrane

The low-molecular-weight compound 118 which had been synthesized above in B1 was bound to bovine serum albumin (BSA) in a manner similar to that described above in B1, and the resultant product was purified through dialysis. The thus-obtained product was employed as a low-molecular-weight compound-protein conjugate to be immobilized onto an unbound labeled antibody capturing section of a chromatomembrane.

### B4. Preparation of chromatomembrane

The low-molecular-weight compound 118-BSA conjugate which had been formed above in B3, which constitutes an unbound labeled antibody capturing section, was applied to Hi-Flow Plus Membrane (HF90, 25 mm × 30 cm, Millipore) at a position 5 mm away from the lower end thereof. Meanwhile, anti-mouse IgG rabbit antibody (Zymed), which constitutes a detection section, was applied to the membrane at a position 15 mm away from the lower end thereof. The solutions of the conjugate and antibody were applied, by use of XYZ Handling System (BioDot), to the membrane so as to form lines. After completion of application, the membrane was allowed to stand at room temperature overnight for drying, and then was employed as a chromatomembrane.

### B5. Preparation of gold-colloidal-particles-labeled anti-Co-PCB monoclonal antibody

Gold colloidal particles (average particle size: about 40 nm, BBInternational) were bound to the anti-Co-PCB monoclonal antibody prepared above in B2 according to the protocol by BBInternational. The OD520 of the resultant gold-colloid-labeled anti-Co-PCB monoclonal antibody solution was regulated to 5 to 6, and the solution was refrigerated until employed.

### B6. Assembly of conjugate-pad-mounted detection instrument

A glass fiber-made conjugate pad (10 mm × 30 cm, product of Millipore) was impregnated with the above-prepared gold-colloid-labeled antibody solution (final concentration: 16%), and then completely dried by use of a vacuum drying machine. The resultant pad was employed as a gold colloid pad. Separately, a cotton pad (20 mm × 30 cm, product of Millipore) was impregnated with a PBS solution containing 2% sucrose, 1% BSA, and 1% Triton X-100, and then completely dried by use of a vacuum drying machine. The resultant pad was employed as a sample pad. These pads and the chromatomembrane prepared above in B4 were mounted on a laminate card (product of Millipore). Furthermore, an absorption pad (17 mm × 30 cm, product of Millipore) was mounted on the card, and subsequently, the resultant card was cut into pieces having a width of 5 mm by use of a guillotine cutter (product of BioDot). Each of the pieces was employed as an immunochromatography instrument.

### B7. Preparation of labeled antibody and detection instrument employed in the present use method (the present detection set)

A mixture (100 µl) of the gold-colloid-labeled antibody solution prepared above in B5 (final concentration: 1%) and a sucrose solution (final concentration: 3%) was added to a 1.5-ml plastic-made Eppendorf tube, and then completely dried by use of a vacuum drying machine. The resultant product was employed as a dried, labeled antibody. A glass fiber filter (Schleicher & Schuell), serving as a reaction product contact section, and the chromatomembrane prepared above in B4 were mounted on a laminate card (product of Millipore). Furthermore, an absorption pad (17 mm × 30 cm, product of Millipore) was mounted on the card, and subsequently, the resultant card was cut into pieces having a width of 5 mm by use of a guillotine cutter (product of BioDot). Each of the pieces was employed as an immunochromatography instrument.

### B8. Detection of Co-PCB #118 by use of conjugate-pad-mounted detection instrument A

Co-PCB #118 was diluted with a TBS solution containing 20% DMSO so that the Co-PCB #118 concentration became 200 ppm, 20 ppm, 2 ppm, 0.2 ppm, or 0.02 ppm. The thus-diluted Co-PCB #118 sample (150 µL) was added dropwise to the test sample application section of the detection instrument. The degree of coloring of the detection section was evaluated through visual observation at different points in time (see Table 2) after addition of the sample. The results are shown in Table 2.

**Table 2**

| Co-PCB 118 concentration (ppm) | Degree of coloring of detection section | | | |
|---|---|---|---|---|
| | 3 minutes later | 5 minutes later | 10 minutes later | 20 minutes later |
| 200 | - | - | + | ++ |
| 20 | - | - | + | ++ |
| 2 | - | - | + | + |
| 0.2 | - | - | - | - |
| 0.02 | - | - | - | - |
| 0 | - | - | - | - |
| [Evaluation criteria] ++: high coloring degree, +: low coloring degree, -: no coloring | | | | |

### B9. Detection of Co-PCB #118 by use of the present detection instrument

Co-PCB #118 was diluted with a TBS solution containing 20% DMSO so that the Co-PCB #118 concentration became 200 ppm, 20 ppm, 2 ppm, 0.2 ppm, or 0.02 ppm. The thus-diluted Co-PCB #118 solution (150 µL) was completely mixed with the dried, labeled antibody obtained above in B7, and the resultant mixture was added dropwise to the reaction product contact section of the present detection instrument prepared above in B7. The degree of coloring of the detection section was evaluated through visual observation at different points in time (see Table 3) after addition of the mixture. The results are shown in Table 3.

**Table 3**

| Co-PCB 118 concentration (ppm) | Degree of coloring of detection section | | | |
|---|---|---|---|---|
| | 3 minutes later | 5 minutes later | 10 minutes later | 20 minutes later |
| 200 | - | + | ++ | ++ |
| 20 | - | + | ++ | ++ |
| 2 | - | + | ++ | + |
| 0.2 | - | - | + | + |
| 0.02 | - | - | - | - |
| 0 | - | - | - | - |
| [Evaluation criteria] ++: high coloring degree, +: low coloring degree, -: no coloring | | | | |

### B10. Results

In the case where the conjugate-pad-mounted detection instrument A was employed, Co-PCB #118 of 2 ppm or more was determined to be positive through visual evaluation 20 minutes after addition of the Co-PCB #118 sample to the detection instrument A. In contrast, in the case where the present use method was performed by use of the present detection instrument B, Co-PCB #118 of 0.2 ppm or more was determined to be positive; i.e., the detection sensitivity of the instrument B was found to be higher by a factor of 10 or more than that of the conjugate-pad-mounted detection instrument A, which is of a conventional type.

### B11. Semi-quantification of Co-PCB #118

In both the cases of the present detection instrument B and the conventional-type conjugate-pad-mounted detection instrument A, Co-PCB #118, which is a target substance, was semi-quantified; i.e., the level of the target substance present in the test sample was determined, by use, as indexes, of the degree of coloring of the detection section and the degree of coloring of the unbound labeled antibody capturing section. Specifically, in the case of the conjugate-pad-mounted detection instrument A, data on the degree of coloring of the unbound labeled antibody capturing section were added to the data on the degree of coloring of the detection section shown in Table 2. The results are shown in Table 4.

**Table 4**

| Degree of coloring of detection section (right) | | | | | | | | |
|---|---|---|---|---|---|---|---|---|
| Co-PCB 118 concentration (ppm) | Degree of coloring of unbound labeled antibody capturing section (left) | | | | | | | |
| | 3 minutes later | | 5 minutes later | | 10 minutes later | | 20 minutes later | |
| 200 | - | - | - | - | - | + | - | ++ |
| 20 | - | - | - | - | - | + | - | ++ |
| 2 | - | - | + | - | + | + | + | + |
| 0.2 | - | - | + | - | + | - | ++ | - |
| 0.02 | - | - | + | - | ++ | - | ++ | - |
| 0 | - | - | + | - | ++ | - | ++ | - |
| [Evaluation criteria] ++: high coloring degree, +: low coloring degree, -: no coloring | | | | | | | | |

As is clear from Table 4, in the case of the conjugate-pad-mounted detection instrument A, 1) when low degree of coloring or no coloring of the unbound labeled antibody capturing section is observed, and when high degree of coloring of the detection section is observed, the Co-PCB 118 concentration is 20 ppm or more; 2) when coloring of the unbound labeled antibody capturing section and coloring of the detection section are observed, the Co-PCB 118 concentration is 2 to 20 ppm; and 3) when high degree of coloring of the unbound labeled antibody capturing section and no coloring of the detection section are observed, the Co-PCB 118 concentration is ppm or less.

Meanwhile, in the case of the present detection instrument B, data on the degree of coloring of the unbound labeled antibody capturing section were added to the data on the degree of coloring of the detection section shown in Table 3. The results are shown in Table 5.

**Table 5**

| Degree of coloring of detection section (right) | | | | | | | | |
|---|---|---|---|---|---|---|---|---|
| Co-PCB 118 concentration (ppm) | Degree of coloring of unbound labeled antibody capturing section (left) | | | | | | | |
| | 3 minutes later | | 5 minutes later | | 10 minutes later | | 20 minutes later | |
| 200 | - | - | - | + | - | ++ | - | ++ |
| 20 | - | - | - | + | - | ++ | - | ++ |
| 2 | - | - | + | + | + | ++ | + | + |
| 0.2 | - | - | + | - | + | + | ++ | - |
| 0.02 | - | - | + | - | ++ | - | ++ | - |
| 0 | - | - | + | - | ++ | - | ++ | - |
| [Evaluation criteria] ++: high coloring degree, +: low coloring degree, -: no coloring | | | | | | | | |

As is clear from Table 5, 1) when very low degree of coloring or no coloring of the unbound labeled antibody capturing section is observed, and when high degree of coloring of the detection section is observed, the Co-PCB 118 concentration is 20 ppm or more; 2) when low degree of coloring of the unbound labeled antibody capturing section and high degree of coloring of the detection section are observed, the Co-PCB 118 concentration is 2 to 20 ppm; 3) when high degree of coloring of the unbound labeled antibody capturing section and low degree of coloring of the detection section are observed, the Co-PCB 118 concentration is 0.2 to 2 ppm; and 4) when high degree of coloring of the unbound labeled antibody capturing section and no coloring of the detection section are observed, the Co-PCB 118 concentration is 0.2 ppm or less.

### Industrial Applicability

The present invention provides means for conveniently and sensitively detecting a low-molecular-weight substance such as an environmental pollutant (e.g., a dioxin), which is a target substance contained in a test sample, which means enables suppression of, for example, false-positive reaction.

## Claims

1. A low-molecular-weight substance detection instrument employing immunochromatography, comprising a test sample application section with which a test sample is brought into contact, wherein a target substance contained in the test sample brought into contact with the test sample application section is detected by use, as an index, of a labeling substance employed in a label product containing, as a portion thereof, an antibody capable of binding to the target substance contained in the test sample.

2. A low-molecular-weight substance detection instrument according to claim 1, which comprises 1) a test sample application section with which a test sample is brought into contact; 2) a label product reaction section containing a label product containing, as a portion thereof, an antibody capable of binding to a target substance contained in the test sample, the label product being not bound to the reaction section; 3) an unbound label product capturing section containing an element capable of capturing the label product which is not bound to the target substance, the element being bound to the capturing section; and 4) a detection section containing a detection element which, when coming into contact with the target substance bound to the label product, causes a visually observable change.

3. A low-molecular-weight substance detection instrument according to claim 1, wherein a test sample is reacted with a labeled antibody containing, as a portion thereof, an antibody capable of binding to a target substance contained in the test sample, and the resultant reaction product is employed for detecting the target substance contained in the test sample.

4. A low-molecular-weight substance detection instrument according to claim 3, which comprises the following 1) and 2):
1) an unbound labeled antibody capturing section containing an element capable of capturing the labeled antibody which is not bound to the target substance, the element being bound to the capturing section; and
2) a detection section containing a detection element which, when coming into contact with the target substance bound to the labeled antibody, causes a visually observable change.

5. A low-molecular-weight substance detection instrument according to claim 2 or 4, wherein the detection element contained in the detection section is metallic colloidal particles or latex particles.

6. A low-molecular-weight substance detection instrument according to claim 2 or 4, wherein the detection element contained in the detection section is bound to the detection section.

7. A low-molecular-weight substance detection instrument according to claim 2 or 4, wherein the element capable of capturing the label product which is not bound to the target substance, which is contained in the unbound label product capturing section, is the target substance or a substance similar to the target substance.

8. A low-molecular-weight substance detection instrument according to claim 2 or 4, wherein each of the unbound label product capturing section and the detection section comprises, as a base, a carrier fixated onto a porous membrane for chromatography.

9. A low-molecular-weight substance detection instrument according to any of claims 1 through 8, wherein the target substance is a dioxin and/or a PCB.

10. A method of using a low-molecular-weight substance detection instrument as recited in claim 3, which comprises bringing, into contact with the test sample application section, a reaction product formed from a test sample and a labeled antibody containing, as a portion thereof, an antibody capable of binding to a target substance contained in the test sample; and detecting a complex of the labeled antibody and the target substance, the complex being contained in the reaction product, and/or the labeled antibody which is not bound to the target substance, to thereby detect the target substance contained in the test sample.

11. A low-molecular-weight substance detection set comprising a low-molecular-weight substance detection instrument as recited in any of claims 1 through 9, and a labeled antibody containing, as a portion thereof, an antibody capable of binding to a target substance contained in a test sample.

12. A low-molecular-weight substance detection set according to claim 11, wherein the labeled antibody containing, as a portion thereof, an antibody capable of binding to a target substance contained in a test sample is maintained in a dry condition.
